# EUROPÄISCHE PATENTSCHRIFT

(11) **EP 3 943 024 B1**
(45) Veröffentlichungstag und Bekanntmachung des Hinweises auf die Patenterteilung: **28.09.2022**
(21) Anmeldenummer: 21181407.4
(22) Anmeldetag: 24.06.2021
(51) Int. Cl.: A61B 17/29

(54) **MEDIZINISCHES INSTRUMENT**
MEDICAL INSTRUMENT
INSTRUMENT MÉDICAL

(30) Priorität: 23.07.2020 DE 102020119462
(43) Veröffentlichungstag der Anmeldung: 26.01.2022
(73) Patentinhaber: Karl Storz SE & Co. KG, 78532 Tuttlingen (DE)
(72) Erfinder: Stefan, Jochen, 78532 Tuttlingen (DE); Kärcher, Daniel, 78532 Tuttlingen (DE)
(74) Vertreter: Hofmeister, Frank

(56) Entgegenhaltungen:
- EP-A1- 3 175 802
- US-A1- 2008 147 113
- US-A1- 2010 331 857

## Beschreibung

Die Erfindung betrifft ein medizinisches Instrument mit einem hohlen Schaft, an dessen proximalem Ende eine Handhabe angeordnet ist und an dessen distalem Ende ein Werkzeug mit zwei Maulteilen angeordnet ist, wobei mindestens ein Maulteil relativ zu dem anderen Maulteil verschwenkbar ausgebildet ist, wobei ein das Werkzeug tragender distaler Endbereich des Schaftes als gegenüber der Längsachse des Schaftes abwinkelbare Werkzeugspitze ausgebildet ist und wobei das Abwinkeln der Werkzeugspitze über ein axialverschiebbar im hohlen Schaft gelagertes und proximalseitig mit der Handhabe in Wirkverbindung stehendes erstes Betätigungselement erfolgt und wobei das mindestens eine verschwenkbare Maulteil des Werkzeugs über ein axialverschiebbar im hohlen Schaft gelagertes und proximalseitig mit der Handhabe in Wirkverbindung stehendes zweites Betätigungselement zwischen einer geschlossenen und eine geöffneten Position verstellbar ist wobei das axialverschiebbare erste Betätigungselement zum Abwinkeln der Werkzeugspitze und das axialverschiebbare zweite Betätigungselement zum Betätigen des mindestens einen verschwenkbaren Maulteils des Werkzeugs in Richtung der Längsachse des Schaftes parallel zueinander angeordnet sind. Medizinische Instrumente für die endoskopische Chirurgie weisen in der Regel einem hohlen Schaft auf, an dessen proximalem Ende eine Handhabe und an dessen distalem Ende ein Werkzeug aus zwei relativ zueinander bewegbaren Maulteilen angeordnet ist. Das als Greif-, Halte- und/oder Schneidinstrument ausgebildete Werkzeug ist über die Handhabe betätigbar. Um bei den häufig beengten Arbeitsverhältnissen mit dem Werkzeug einen möglichst großen Wirkungsbereich abdecken zu können, sind viele endoskopische Instrumente so ausgebildet, dass das Werkzeug gegenüber der Längsachse des Schaftes abwinkelbar ausgebildet ist sowie das Werkzeug um die Längsachse des Schaftes drehbar ist.

Ein gattungsgemäßes medizinisches Instrument ist beispielsweise aus der EP 3 175 802 A1 bekannt. Dieses bekannte medizinische Instrument hat sich in der Praxis bewährt, jedoch kann es aufgrund der relativ zur Instrumentenlängsachse exzentrischen Lagerung der Betätigungselemente zum Abwinkeln der Werkzeugspitze und zum Betätigen des verschwenkbaren Maulteils bei hoher Kraftbelastung der Maulteile dazu kommen, dass sich die beiden Betätigungselemente durchbiegen und im Knickbereich zur Werkzeugspitze über den Durchmesser des Instrumentenschaftes hinaustreten, was insbesondere bei der Verwendung eines Trokars problematisch sein kann.

Aus der US 2010/0331857 A1 ist ein flexibles Gelenk zum Abwinkeln einer Werkzeugspitze bekannt. Dieser bekannte Abwinklungsmechanismus besteht aus einer Reihe hintereinander angeordneter kardanischer Gelenke, über die die Werkzeugspitze relativ zur Instrumentenlängsachse abwinkelbar ist. Insbesondere bei einer rechtwinkligen Abwinklung der Werkzeugspitze treten Teile des Abwinklungsmechanismus im Knickbereich über den Durchmesser des Instrumentenschaftes hinaus, was insbesondere bei der Verwendung eines Trokars problematisch sein kann.

Eine weitere Gelenkvorrichtung ist aus der US 2008/0147113 A1 bekannt. Auch bei diesem bekannten Gelenksystem treten bei der Abwinklung der Werkzeugspitze einzelne Bauteile der Gelenkvorrichtung über den Durchmesser des Instrumentenschaftes hinaus, was insbesondere bei der Verwendung eines Trokars problematisch sein kann.

Davon ausgehend liegt der Erfindung die **Aufgabe** zugrunde, ein medizinisches Instrument der eingangs genannten Art so auszugestalten, dass auch bei abgewinkelter Werkzeugspitze ein stets gleichbleibender Außendurchmesser des Instrumentenschaftes gewährleistet ist.

Die **Lösung** dieser Aufgabenstellung ist erfindungsgemäß dadurch gekennzeichnet, dass das axialverschiebbare erste Betätigungselement zum Abwinkeln der Werkzeugspitze und das axialverschiebbare zweite Betätigungselement zum Betätigen des mindestens einen verschwenkbaren Maulteils des Werkzeugs in axialer Richtung der Längsachse des Schaftes führend aneinander gelagert sind sowie mindestens eines der beiden Betätigungselemente zusätzlich führend an der Innenseite des distalen Endes des Schaftes gelagert ist, wobei die gegenseitige axiale Lagerung der Betätigungselemente aneinander sowie an der Innenseite des distalen Endes des Schaftes über eine Zapfen-Schlitz-Steuerung erfolgt.

Durch die erfindungsgemäße gegenseitige Führung der axialverschiebbaren Betätigungselemente aneinander bei gleichzeitiger Führung zumindest eines der Betätigungselemente auch an der Innenseite des Schaftes wird aufgrund der Ausbildung der gegenseitigen axialen Lagerungen als Zapfen-Schlitz-Steuerungen sichergestellt, dass die Betätigungselemente auch bei hoher Kraftbelastung im Abknickbereich der Werkzeugspitze nicht über den Außendurchmesser des Instrumentenschaftes heraustreten können, da durch die erfindungsgemäße Konstruktion eine gegenseitige Hemmung zum Ausweichen in eine radiale Richtung erfolgt. Die Ausbildung der gegenseitigen Führungen als Zapfen-Schlitz-Steuerungen stellt dabei eine einfach und sicher zu handhabende Führung bei gleichzeitig axialer Beweglichkeit der miteinander gekoppelten Bauteile dar.

Zur Ausgestaltung der Zapfen-Schlitz-Steuerung wird mit einer praktischen Ausführungsform der Erfindung vorgeschlagen, dass die Zapfen-Schlitz-Steuerung als an zumindest einem der aneinander gelagerten Bauteile, der Betätigungselemente oder der Innenseite des distalen Endes des Schaftes, ausgebildete Führungsnut und am jeweils korrespondierenden anderen Bauteil ausgebildeter Führungssteg ausgebildet ist, wobei der Führungssteg führend in der korrespondierenden Führungsnut Aufnahme findet.

Gemäß einer weiteren Ausführungsform der Erfindung wird vorgeschlagen, dass zusätzlich zu dem axialverschiebbaren ersten Betätigungselement zum Abwinkeln der Werkzeugspitze und dem axialverschiebbaren zweiten Betätigungselement zum Betätigen des mindestens einen verschwenkbaren Maulteils des Werkzeugs ein drehbar gelagertes drittes Betätigungselement zum Verdrehen des Werkzeugs um die Längsachse des Schaftes im hohlen Schaft angeordnet ist.

Weiterhin wird mit der Erfindung vorgeschlagen, dass zusätzlich zu dem axialverschiebbaren ersten Betätigungselement zum Abwinkeln der Werkzeugspitze und dem axialverschiebbaren zweiten Betätigungselement zum Betätigen des mindestens einen verschwenkbaren Maulteils des Werkzeugs sowie dem dritten Betätigungselement zum Verdrehen des Werkzeugs um die Längsachse des Schaftes ein axialverschiebbares viertes Betätigungselement zum Lösen einer Maulteilraste im Schaft angeordnet ist. Mittels der Maulteilraste ist es möglich, die Maulteile in einer bestimmten Position zueinander zu fixieren, um den Operateur zu entlasten. Um sicherzustellen, dass auch das zusätzliche vierte Betätigungselement zum Lösen der Maulteilraste nicht über den Außendurchmesser des Instrumentenschaftes heraustreten kann, wird erfindungsgemäß vorgeschlagen, dass das zusätzliche axialverschiebbare vierte Betätigungselement zum Lösen der Maulteilraste ebenfalls führend an den führend aneinander gelagerten Bauteilen, der Betätigungselemente oder der Innenseite des distalen Endes des Schaftes, gelagert ist.

Gemäß einer praktischen Ausführungsform zur Lagerung der verschiedenen Betätigungselemente innerhalb des Instrumentenschaftes wird mit der Erfindung vorgeschlagen, dass das erste Betätigungselement zum Abwinkeln der Werkzeugspitze mittig zwischen dem zweiten Betätigungselement zum Betätigen des mindestens einen verschwenkbaren Maulteils und dem vierten Betätigungselement zum Lösen der Maulteilraste angeordnet ist.

Zur Praktischen Ausbildung der gegenseitigen Führungen der miteinander gekoppelten Betätigungselemente wird erfindungsgemäß vorgeschlagen, dass zur Ausbildung der Zapfen-Schlitz-Steuerung am ersten Betätigungselement zum Abwinkeln der Werkzeugspitze zwei um 180° versetzt zueinander angeordnete Führungsstege angeordnet sind, die in korrespondierende Führungsnuten eingreifen, die am zweiten Betätigungselement zum Betätigen des mindestens einen verschwenkbaren Maulteils und am vierten Betätigungselement zum Lösen der Maulteilraste ausgebildet sind und, dass an den der Innenseite des distalen Endes des Schaftes zugewandten Seiten des zweiten Betätigungselements zum Betätigen des mindestens einen verschwenkbaren Maulteils und des vierten Betätigungselements zum Lösen der Maulteilraste jeweils ein Führungssteg angeordnet ist, der in eine korrespondierende Führungsnut eingreift, die an der Innenseite des distalen Endes des Schaftes ausgebildet ist.

Weiterhin wird mit der Erfindung vorgeschlagen, dass ein proximalseitig hinter der Zapfen-Schlitz-Steuerung angeordneter Schaft des ersten Betätigungselements zum Abwinkeln der Werkzeugspitze im Querschnitt kreisförmig ausgebildet ist und die parallel verlaufenden Schäfte des zweiten Betätigungselements zum Betätigen des mindestens einen verschwenkbaren Maulteils sowie des vierten Betätigungselements zum Lösen der Maulteilraste im Querschnitt jeweils halbkreisförmig so ausgebildet sind, dass die im Querschnitt halbkreisförmigen Schäfte den Schaft des ersten Betätigungselements zum Abwinkeln der Werkzeugspitze im zusammengesetzten Zustand koaxial umgeben. Die teilweise koaxiale Lagerung der Betätigungselemente zueinander ermöglicht einerseits eine zusätzliche gegenseitige Führung und Stabilisierung stellt andererseits eine besonders platzsparende Anordnung der Betätigungselemente innerhalb des Instrumentenschaftes dar.

Um die axiale Verschiebbarkeit der Betätigungselement bei gleichzeitiger gegenseitiger Führung zu gewährleisten, wird mit der Erfindung vorgeschlagen, dass die Führungsnuten in Axialrichtung um den Verschiebeweg der in die jeweiligen Führungsnuten eingreifenden korrespondierenden Führungsstege länger ausgebildet sind, als die jeweiligen Führungsstege.

Schließlich wird mit der Erfindung vorgeschlagen, dass der Verschiebeweg der jeweiligen Führungsstege innerhalb der korrespondierenden Führungsnuten über die axiale Länge der jeweiligen Führungsnuten begrenzbar ist.

Weitere Merkmale und Vorteile der Erfindung ergeben sich anhand der zugehörigen Zeichnungen, in denen ein Ausführungsbeispiel eines erfindungsgemäßen medizinischen Instruments nur beispielhaft dargestellt ist, ohne die Erfindung auf dieses Ausführungsbeispiel zu beschränken. In den Zeichnungen zeigt:
- Fig. 1: eine perspektivische Seitenansicht eines erfindungsgemäßen medizinischen Instruments;
- Fig. 2: eine vergrößerte Ansicht des Details II gemäß Fig. 1, gegenüber Fig. 1 um 90° um die Längsachse des medizinischen Instruments gedreht;
- Fig. 3: eine Explosionszeichnung des Details III gemäß Fig. 2;
- Fig. 4: eine Ansicht gemäß Fig. 3, jedoch um 90° gedreht;
- Fig. 5: eine erste vergrößerte Detailansicht aus den Abbildungen Fig. 3 und 4;
- Fig. 6: eine zweite vergrößerte Detailansicht aus den Abbildungen Fig. 3 und 4 und
- Fig. 7: eine schematische Darstellung einer Maulteilraste.

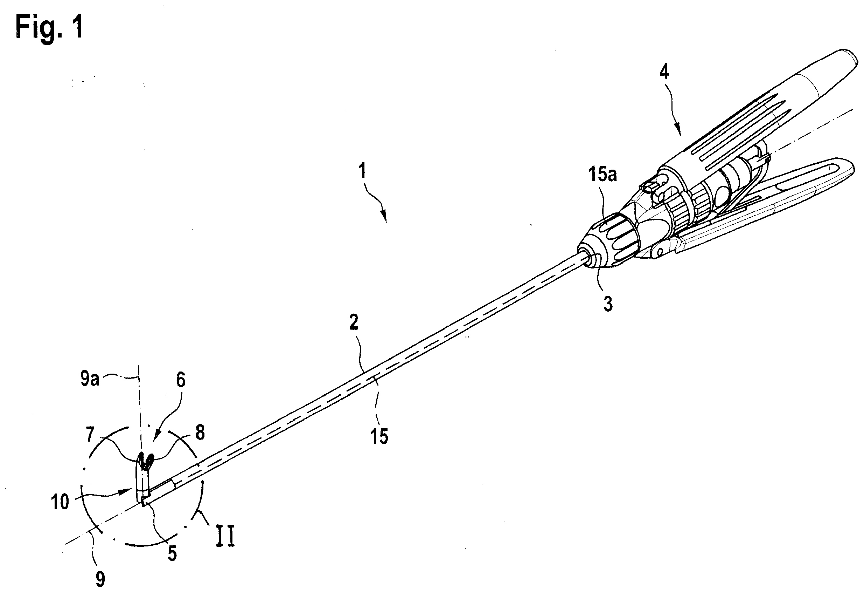

Die Abbildung Fig. 1 zeigt ein medizinisches Instrument 1 mit einem hohlen Schaft 2, an dessen proximalem Ende 3 eine Handhabe 4 angeordnet ist und an dessen distalem Ende 5 ein Werkzeug 6 angeordnet ist, das bei der dargestellten Ausführungsform aus einem starren Maulteil 7 und einem gegenüber dem starren Maulteil 7 verschwenkbaren Maulteil 8 besteht.

Gemäß einer alternativen Ausführungsform ist es selbstverständlich auch möglich, beide Maulteile 7 und 8 relativ zueinander verschwenkbar auszugestalten.

Um dem Werkzeug 6 möglichst viele Freiheitsgrade zur Bewegung relativ zum Schaft 2 einzuräumen, ist ein das Werkzeug 6 tragender distaler Endbereich des Schaftes 2 als gegenüber der Längsachse 9 des Schaftes 2 abwinkelbare Werkzeugspitze 10 ausgebildet. In der Darstellung gemäß Fig. 1 ist die Werkzeugspitze 10 gegenüber der Längsachse 9 des Schaftes 2 etwa um 90° abgewinkelt.

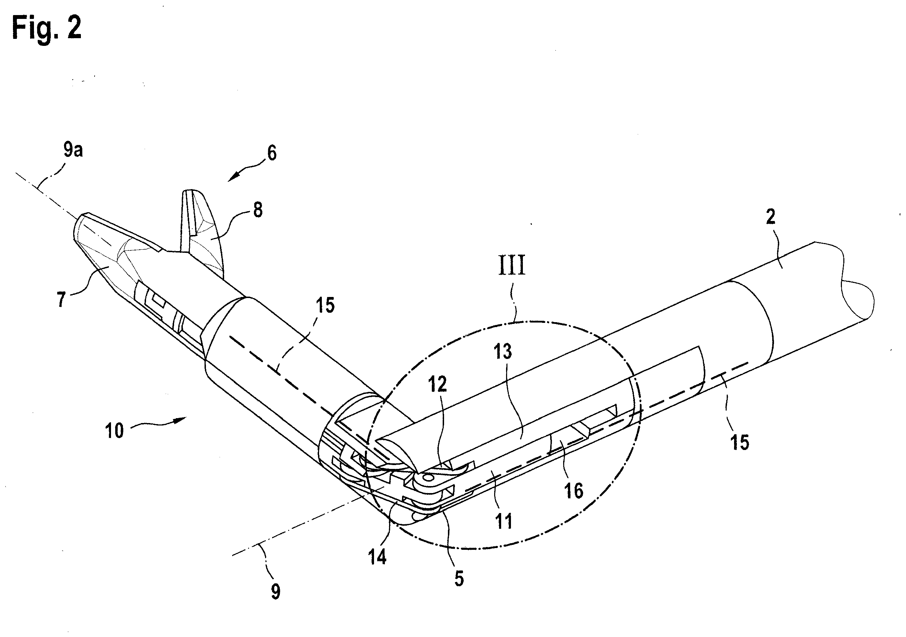

Das Abwinkeln der Werkzeugspitze 10 erfolgt, wie aus den Abbildungen Fig. 2 bis 4 ersichtlich, über ein axialverschiebbar im hohlen Schaft 2 gelagertes und proximalseitig mit der Handhabe 4 in Wirkverbindung stehendes erstes Betätigungselement 11, das als Zug-/Druckstange ausgebildet ist. Mit der Werkzeugspitze 10 ist das erste Betätigungselement 11 über einen Anlenkhebel 12 verbunden, der proximalseitig gelenkig am ersten Betätigungselement 11 und distalseitig gelenkig an der Werkzeugspitze 10 gelagert ist.

Das Verstellen des verschwenkbaren Maulteils 8 des Werkzeugs 6 zwischen einer geschlossenen und eine geöffneten Position erfolgt, wie aus den Abbildungen Fig. 2 bis 4 ersichtlich, über ein axialverschiebbar im hohlen Schaft 2 gelagertes und proximalseitig mit der Handhabe 4 in Wirkverbindung stehendes zweites Betätigungselement 13, das ebenfalls als Zug-/Druckstange ausgebildet ist. Mit dem verschwenkbaren Maulteil 8 ist das zweite Betätigungselement 13 über einen Anlenkhebel 14 verbunden, der proximalseitig gelenkig am zweiten Betätigungselement 13 und distalseitig mit dem verschwenkbaren Maulteil 8 in Wirkverbindung steht.

Weiterhin ist das Werkzeug 6 um die Längsachse 9 des Schaftes 2 bzw. bei abgewinkelter Werkzeugspitze 10 um die Längsachse 9a der Werkzeugspitze 10 drehbar, wobei das Verdrehen des Werkzeugs 6 um die Längsachse 9 des Schaftes 2 über ein in dem hohlen Schaft 2 drehbar gelagertes drittes Betätigungselement 15 erfolgt, das proximalseitig mit der Handhabe 4 in Wirkverbindung steht, wobei das dritte Betätigungselement 15 zweiteilig ausgebildet aus einem in der abwinkelbaren Werkzeugspitze 10 gelagerten distalen Teilbereich sowie einem im proximalen Teil des Schaftes 2 gelagerten proximalen Teilbereich besteht.

Zur Ausbildung des dritten Betätigungselements 15 kann für dieses sowohl eine massive Stange als auch ein Hohlrohr verwendet werden.

Die beiden einander zugewandten Stirnseiten der proximalen und distalen Teilbereiche des dritten Betätigungselements 15 stehen im Übergang zur abwinkelbaren Werkzeugspitze10 beispielsweise über nicht dargestellte Stirnverzahnungen miteinander in Eingriff. Die Stirnverzahnungen übertragen die Rotation des proximalen Teilbereichs des dritten Betätigungselements 15 um die Längsachse 9 des Schaftes 2 auf den distalen Teilbereich des dritten Betätigungselements 15 zur Rotation um die Längsachse 9a der Werkzeugspitze 10.

Zusätzlich zu dieser Rotation des Werkzeugs 6 um die Längsachse 9 des Schaftes 2 bzw. bei abgewinkelter Werkzeugspitze 10 um die Längsachse 9a der Werkzeugspitze 10 mittels des dritten Betätigungselements 15 ist der Schaft 3 über einen an der Handhabe 4 angeordneten Drehantrieb 15a um die Längsachse 9 des Schaftes 2 verdrehbar.

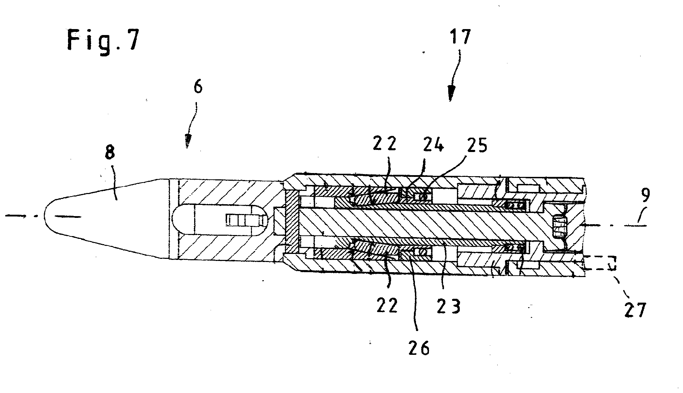

Zusätzlich zu dem axialverschiebbar im Schaft 2 gelagerten ersten Betätigungselement 11 zum Abwinkeln der Werkzeugspitze 10 sowie dem zweiten Betätigungselement 13 zum Betätigen des verschwenkbaren Maulteils 8 des Werkzeugs 6 weist das dargestellte medizinische Instrument 1 ein viertes Betätigungselement 16 auf, das axialverschiebbar im Schaft 2 gelagert dazu dient, eine am Werkzeug 6 ausgebildete Maulteilraste 17 zu lösen. Mittels der in Fig. 7 schematisch dargestellten Maulteilraste 17 ist es möglich, die Maulteile 7 und 8 in einer bestimmten Position zueinander, insbesondere in der geschlossenen Position der Maulteile 7 und 8, zu fixieren, um den Operateur zu entlasten. Das vierte Betätigungselement 16 ist ebenfalls als Zug-/Druckstange ausgebildet.

Zur platzsparenden Lagerung der verschiedenen Betätigungselemente innerhalb des Instrumentenschaftes 2 ist das erste Betätigungselement 11 zum Abwinkeln der Werkzeugspitze 10 mittig zwischen dem zweiten Betätigungselement 13 zum Betätigen des verschwenkbaren Maulteils 8 und dem vierten Betätigungselement 16 zum Lösen der Maulteilraste 17 angeordnet.

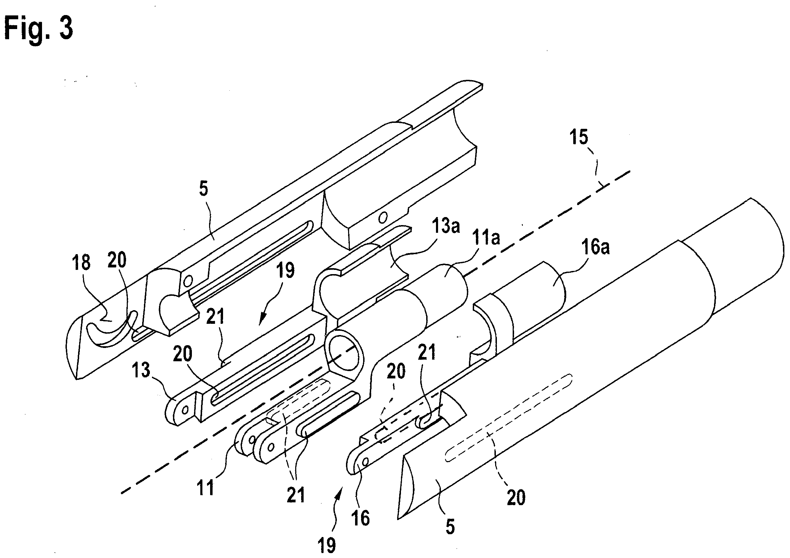

Um zu vermeiden, dass sich die Betätigungselemente 11, 13 und 16 bei hoher Kraftbelastung der Maulteile 7 und 8 durchbiegen und im Knickbereich vom Schaft 2 zur Werkzeugspitze 10 über den Durchmesser des Instrumentenschaftes 2 hinaustreten, was insbesondere bei der Verwendung eines Trokars problematisch sein kann, sind zumindest einige der Betätigungselemente 11, 13 und 16 in Richtung der Längsachse 9 des Schaftes 2 parallel zueinander angeordnet und in axialer Richtung der Längsachse 9 des Schaftes 2 führend aneinander gelagert sowie zumindest teilweise zusätzlich führend an der Innenseite 18 des distalen Endes 5 des Schaftes 2 gelagert ist, wie dies den Abbildungen Fig. 3 bis 6 zu entnehmen ist.

Diese Konstruktion mit der führenden Lagerung der Betätigungselemente 11, 13 und 16 aneinander sowie an der Innenseite 18 des Schaftes 2 bewirkt eine gegenseitige Hemmung, die ein Durchbiegen in eine radiale Richtung verhindert.

Wie aus den Abbildungen Fig. 3 bis 6 ersichtlich, erfolgt die gegenseitige axiale Lagerung der Betätigungselemente 11, 13 und 16 sowie der Innenseite 18 des distalen Endes 5 des Schaftes 2 jeweils über eine Zapfen-Schlitz-Steuerung 19, die an den distalen Endbereichen der Betätigungselemente 11, 13 und 16 und an Innenseite 18 des distalen Endes 5 des Schaftes 2 ausgebildet ist. Die Ausbildung der gegenseitigen Führungen als Zapfen-Schlitz-Steuerungen 19 stellt eine einfach und sicher zu handhabende Führung bei gleichzeitig axialer Beweglichkeit der miteinander gekoppelten Bauteile 11, 13, 16 und 18 dar.

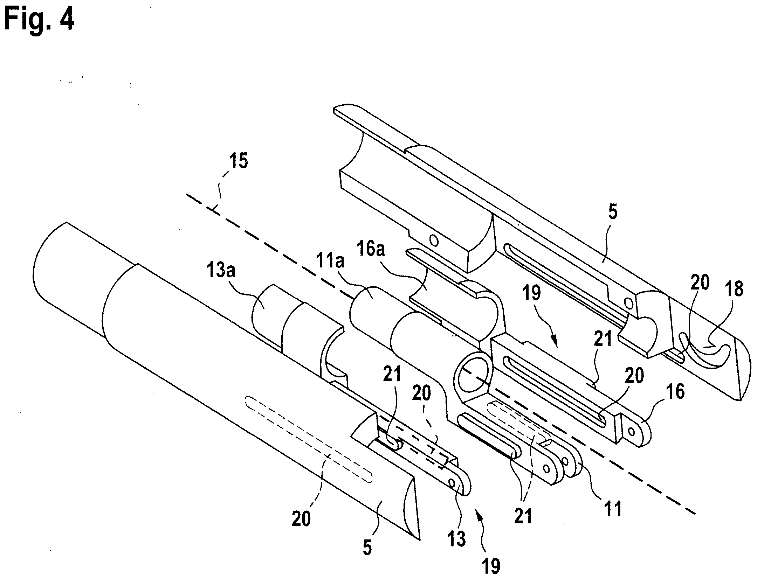

Proximalseitig hinter den Zapfen-Schlitz-Steuerungen 19 sind die Betätigungselemente 11, 13 und 16, wie aus den Abbildungen Fig. 3 und 4 ersichtlich, parallel zueinander so im Inneren des Schaftes 2 gelagert, dass dies eine besonders platzsparende und kompakte Bauweise ermöglicht, um den Außendurchmesser des Schaftes 2 so klein wie möglich zu halten.

Gemäß der dargestellten Ausführungsform ist der proximalseitige Schaft 11a des ersten Betätigungselements 12 zum Abwinkeln der Werkzeugspitze 10 im Querschnitt kreisförmig ausgebildet ist, während die parallel verlaufenden Schäfte 13a und 16a des zweiten Betätigungselements 13 zum Betätigen des verschwenkbaren Maulteils 8 sowie des vierten Betätigungselements 16 zum Lösen der Maulteilraste 17 im Querschnitt jeweils halbkreisförmig so ausgebildet sind, dass die im Querschnitt halbkreisförmigen Schäfte 13a und 16a den Schaft 11a des ersten Betätigungselements 11 zum Abwinkeln der Werkzeugspitze 10 im zusammengesetzten Zustand koaxial umgeben. Der kreisförmige Schaft 11a des ersten Betätigungselements 11 ist darüber hinaus als Hohlrohr ausgebildet. Das freie Lumen des den Schaft 11a des ersten Betätigungselements 11 bildenden Hohlrohres dient zur Aufnahme und führenden Lagerung des dritten Betätigungselements 15 zum Verdrehen des Werkzeugs 6 um die Längsachse 9 des Schaftes 2, wie dies in Fig. 3 und 4 gestrichelt dargestellt ist.

Aufgrund dieser Führung des dritten Betätigungselements 15 innerhalb des Schaftes 11a des ersten Betätigungselements 11 sowie der Zweiteilung des dritten Betätigungselements 15 in einen proximalen und einen distalen Teilbereich bedarf es auf Seiten des dritten Betätigungselements 15 keiner Zapfen-Schlitz-Steuerung 19 oder dergleichen, um ein Durchbiegen im Knickbereich vom Schaft 2 zur Werkzeugspitze 10 zu verhindern.

Die Zapfen-Schlitz-Steuerungen 19 sind als an zumindest einem der aneinander gelagerten Bauteile, der Betätigungselemente 11, 13, 16 oder der Innenseite 18 des distalen Endes 5 des Schaftes 2, ausgebildete Führungsnut 20 und am jeweils korrespondierenden anderen Bauteil ausgebildeter Führungssteg 21 ausgebildet, wobei jeder Führungssteg 21 führende Aufnahme in einer korrespondierenden Führungsnut 20 findet.

Den Abbildungen Fig. 3 bis 6 ist der konstruktive Aufbau der Zapfen-Schlitz-Steuerungen 19 zu entnehmen.

Zur Ausbildung der Zapfen-Schlitz-Steuerung 19 sind am mittig angeordneten ersten Betätigungselement 11 zum Abwinkeln der Werkzeugspitze 10 zwei um 180° versetzt zueinander angeordnete Führungsstege 21 angeordnet, die in korrespondierende Führungsnuten 20 eingreifen, die einerseits am zweiten Betätigungselement 13 zum Betätigen des verschwenkbaren Maulteils 8 und andererseits am vierten Betätigungselement 16 zu Lösen der Maulteilraste 17 ausgebildet sind. Weiterhin ist an den der Innenseite 18 des distalen Endes 5 des Schaftes 2 zugewandten Seiten des zweiten Betätigungselements 13 zum Betätigen des verschwenkbaren Maulteils 8 und des vierten Betätigungselements 16 zu Lösen der Maulteilraste 17 jeweils ein Führungssteg 21 angeordnet, der in eine korrespondierende Führungsnut 20 eingreift, die an der Innenseite 18 des distalen Endes 5 des Schaftes 2 ausgebildet ist.

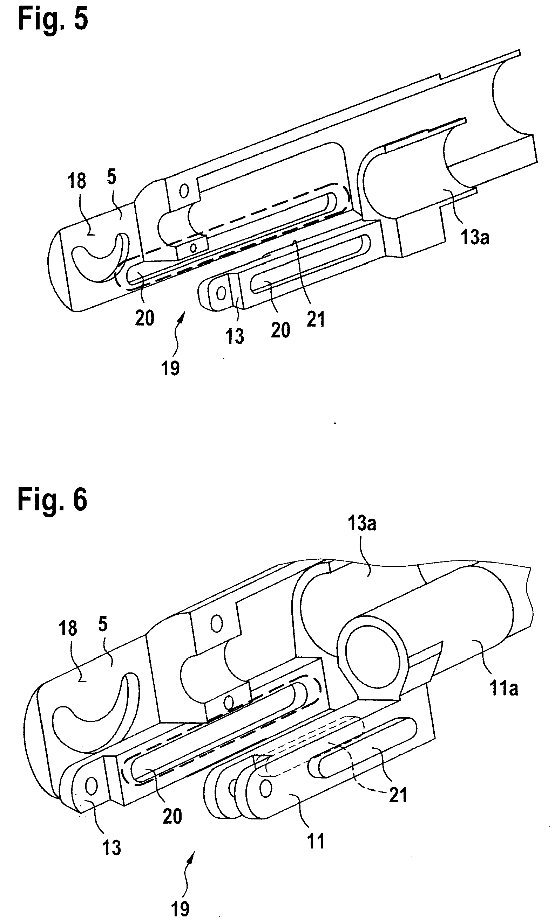

Wie weiterhin insbesondere aus den Abbildungen Fig. 5 und 6 ersichtlich, sind die Führungsnuten 20 in Axialrichtung länger ausgebildet, als die jeweiligen, in den Führungsnuten 20 aufgenommenen Führungsstege 21. Diese längere axiale Erstreckung der Führungsnuten 20 ist erforderlich, um die axiale Verschiebbarkeit der Betätigungselement 11, 13 und 16 bei gleichzeitiger gegenseitiger Führung zu gewährleisten. Aus diesem Grunde sind die Führungsnuten 20 in Axialrichtung um den Verschiebeweg der in die jeweiligen Führungsnuten 20 eingreifenden korrespondierenden Führungsstege 21 länger ausgebildet sind, als die jeweiligen Führungsstege 21.

Ebenso lässt sich der Verschiebeweg der jeweiligen Führungsstege 21 innerhalb der korrespondierenden Führungsnuten 20 über die axiale Länge der jeweiligen Führungsnuten 20 begrenzen.

Die Abbildung Fig. 7 zeigt schematisch den Aufbau der Maulteilraste 17, wie diese aus der DE 10 2016 103 640 A1 bekannt ist.

Über die Maulteilraste 17 lassen sich die Maulteile 7 und 8 in der geschlossenen Position fixieren um beispielsweise eine zwischen den Maulteilen 7 und 8 gehaltene chirurgische Nadel auch ohne weiteres Zutun des Operateurs sicher halten und führen zu können.

Die dargestellte Maulteilraste 17 besteht im Wesentlichen aus zwei symmetrisch zur Längsachse 9 angeordneten und längsverschiebbar in der Werkzeugspitze 10 gelagerten Verriegelungselementen 22, über die die Axialbewegung einer Zughülse 23 blockierbar ist, die zum Betätigen des mindestens einen verschwenkbaren Maulteils 8 distalseitig mit dem mindestens einen verschwenkbaren Maulteil 8 gekoppelt ist.

Die Verriegelungselemente 22 sind mit einem ringförmigen, längsveschiebbar auf der Zughülse 23 gelagerten Verriegelungsschlitten 24 verbunden. Der Verriegelungsschlitten 24 seinerseits ist über ein Drehlager 25 drehbar mit einem Zwischenstück 26 verbunden, das über einen Verbindungshebel 27 mit dem vierten Betätigungselement 16 im Schaft 2 verbunden ist.

Ein wie zuvor beschrieben aufgebautes medizinisches Instrument 1 zeichnet sich dadurch aus, dass aufgrund der gegenseitigen Führung der Betätigungselemente 11, 13 und 16 aneinander sowie an der Innenseite 18 des distalen Endes 5 des Schaftes 2 mittels der als Zapfen-Schlitz-Steuerung 19 ausgebildeten gegenseitigen axiale Lagerung der Betätigungselemente 11 und 13 sowie der Innenseite 18 des distalen Endes 5 des Schaftes 2, ein Durchbiegen der Betätigungselemente 11, 13 und 16 ausgeschlossen ist und somit auch bei abgewinkelter Werkzeugspitze 10 ein stets gleichbleibender der Außendurchmesser des Instrumentenschaftes 2 gewährleistet ist.

### Bezugszeichenliste

- 1: medizinisches Instrument
- 2: Schaft
- 3: proximales Ende (Schaft)
- 4: Handhabe
- 5: distales Ende (Schaft)
- 6: Werkzeug
- 7: starres Maulteil
- 8: verschwenkbares Maulteil
- 9: Längsachse (Schaft)
- 9a: Längsachse (Werkzeugspitze)
- 10: Werkzeugspitze
- 11: erstes Betätigungselement (abwinkeln)
- 11a: Schaft
- 12: Anlenkhebel
- 13: zweites Betätigungselement (Maulteil)
- 13a: Schaft
- 14: Anlenkhebel
- 15: drittes Betätigungselement (drehen)
- 15a: Drehantrieb
- 16: viertes Betätigungselement (Maulteilraste)
- 16a: Schaft
- 17: Maulteilraste
- 18: Innenseite (Schaft)
- 19: Zapfen-Schlitz-Steuerung
- 20: Führungsnut
- 21: Führungssteg
- 22: Verriegelungselement
- 23: Zughülse
- 24: Verriegelungsschlitten
- 25: Drehlager
- 26: Zwischenstück
- 27: Verbindungshebel

## Patentansprüche

1. Medizinisches Instrument (1) mit einem hohlen Schaft (2), an dessen proximalem Ende (3) eine Handhabe (4) angeordnet ist und an dessen distalem Ende (5) ein Werkzeug (6) mit zwei Maulteilen (7, 8) angeordnet ist, wobei mindestens ein Maulteil (8) relativ zu dem anderen Maulteil (7) verschwenkbar ausgebildet ist, wobei ein das Werkzeug (6) tragender distaler Endbereich des Schaftes (2) als gegenüber der Längsachse (9) des Schaftes (2) abwinkelbare Werkzeugspitze (10) ausgebildet ist und wobei das Abwinkeln der Werkzeugspitze (10) über ein axialverschiebbar im hohlen Schaft (2) gelagertes und proximalseitig mit der Handhabe (4) in Wirkverbindung stehendes erstes Betätigungselement (11) erfolgt und wobei das mindestens eine verschwenkbare Maulteil (8) des Werkzeugs (6) über ein axialverschiebbar im hohlen Schaft (2) gelagertes und proximalseitig mit der Handhabe (4) in Wirkverbindung stehendes zweites Betätigungselement (13) zwischen einer geschlossenen und eine geöffneten Position verstellbar ist, wobei das axialverschiebbare erste Betätigungselement (11) zum Abwinkeln der Werkzeugspitze (10) und das axialverschiebbare zweite Betätigungselement (13) zum Betätigen des mindestens einen verschwenkbaren Maulteils (8) des Werkzeugs (6) in Richtung der Längsachse (9) des Schaftes (2) parallel zueinanderangeordnet sind,
**dadurch gekennzeichnet,**
**dass** das axialverschiebbare erste Betätigungselement (11) zum Abwinkeln der Werkzeugspitze (10) und das axialverschiebbare zweite Betätigungselement (13) zum Betätigen des mindestens einen verschwenkbaren Maulteils (8) des Werkzeugs (6) in axialer Richtung der Längsachse (9) des Schaftes (2) führend aneinander gelagert sind sowie mindestens eines der beiden Betätigungselemente (11 oder 13) zusätzlich führend an der Innenseite (18) des distalen Endes (5) des Schaftes (2) gelagert ist, wobei die gegenseitige axiale Lagerung der Betätigungselemente (11 und 13) aneinander sowie an der Innenseite (18) des distalen Endes (5) des Schaftes (2) über eine Zapfen-Schlitz-Steuerung (19) erfolgt.

2. Medizinisches Instrument (1) nach Anspruch 1, **dadurch gekennzeichnet, dass** die Zapfen-Schlitz-Steuerung (19) als an zumindest einem der aneinander gelagerten Bauteile, dem ersten Betätigungselement (11), dem zweiten Betätigungselement (13) oder der Innenseite (18) des distalen Endes (5) des Schaftes (2), ausgebildete Führungsnut (20) und am jeweils korrespondierenden anderen Bauteil (11, 13 oder 18) ausgebildeter Führungssteg (21) ausgebildet ist, wobei der Führungssteg (21) führend in der korrespondierenden Führungsnut (20) Aufnahme findet.

3. Medizinisches Instrument (1) nach Anspruch 1 oder 2, **dadurch gekennzeichnet, dass** zusätzlich zu dem axialverschiebbaren ersten Betätigungselement (11) zum Abwinkeln der Werkzeugspitze (10) und dem axialverschiebbaren zweiten Betätigungselement (13) zum Betätigen des mindestens einen verschwenkbaren Maulteils (8) des Werkzeugs (6) ein drehbar gelagertes drittes Betätigungselement (15) zum Verdrehen des Werkzeugs (6) um die Längsachse (9) des Schaftes (2) im hohlen Schaft (2) angeordnet ist.

4. Medizinisches Instrument (1) nach einem der Ansprüche 1 bis 3, **dadurch gekennzeichnet, dass** zusätzlich zu dem axialverschiebbaren ersten Betätigungselement (11) zum Abwinkeln der Werkzeugspitze (10) und dem axialverschiebbaren zweiten Betätigungselement (13) zum Betätigen des mindestens einen verschwenkbaren Maulteils (8) des Werkzeugs (6) sowie dem dritten Betätigungselement (15) zum Verdrehen des Werkzeugs (6) um die Längsachse (9) des Schaftes (2) ein axialverschiebbares viertes Betätigungselement (16) zum Lösen einer Maulteilraste (17) im Schaft (2) angeordnet ist.

5. Medizinisches Instrument (1) nach Anspruch 4, **dadurch gekennzeichnet, dass** das zusätzliche axialverschiebbare vierte Betätigungselement (16) zum Lösen der Maulteilraste (17) ebenfalls führend an den führend aneinander gelagerten Bauteilen, dem ersten Betätigungselement (11), dem zweiten Betätigungselement (13) oder der Innenseite (18) des distalen Endes (5) des Schaftes (2), gelagert ist.

6. Medizinisches Instrument (1) nach Anspruch 4 oder 5, **dadurch gekennzeichnet, dass** das erste Betätigungselement (11) zum Abwinkeln der Werkzeugspitze (10) mittig zwischen dem zweiten Betätigungselement (13) zum Betätigen des mindestens einen verschwenkbaren Maulteils (8) und dem vierten Betätigungselement (16) zum Lösen der Maulteilraste (17) angeordnet ist.

7. Medizinisches Instrument (1) nach Anspruch 6, **dadurch gekennzeichnet, dass** zur Ausbildung der Zapfen-Schlitz-Steuerung (19) am ersten Betätigungselement (11) zum Abwinkeln der Werkzeugspitze (10) zwei um 180° versetzt zueinander angeordnete Führungsstege (21) angeordnet sind, die in korrespondierende Führungsnuten (20) eingreifen, die am zweiten Betätigungselement (13) zum Betätigen des mindestens einen verschwenkbaren Maulteils (8) und am vierten Betätigungselement (16) zum Lösen der Maulteilraste (17) ausgebildet sind und, dass an den der Innenseite (18) des distalen Endes (5) des Schaftes (2) zugewandten Seiten des zweiten Betätigungselements (13) zum Betätigen des verschwenkbaren Maulteils (8) und des vierten Betätigungselements (16) zum Lösen der Maulteilraste (16) jeweils ein Führungssteg (21) angeordnet ist, der in eine korrespondierende Führungsnut (20) eingreift, die an der Innenseite (18) des distalen Endes (5) des Schaftes (2) ausgebildet ist.

8. Medizinisches Instrument (1) nach Anspruch 6 oder 7, **dadurch gekennzeichnet, dass** ein proximalseitig hinter der Zapfen-Schlitz-Steuerung (19) angeordneter Schaft (11a) des ersten Betätigungselements (11) zum Abwinkeln der Werkzeugspitze (10) im Querschnitt kreisförmig ausgebildet ist und die parallel verlaufenden Schäfte (13a und 16a) des zweiten Betätigungselements (13) zum Betätigen des mindestens einen verschwenkbaren Maulteils (8) sowie des vierten Betätigungselements (16) zum Lösen der Maulteilraste (17) im Querschnitt jeweils halbkreisförmig so ausgebildet sind, dass die im Querschnitt halbkreisförmigen Schäfte (13a und 16a) den Schaft (11a) des ersten Betätigungselements (11) zum Abwinkeln der Werkzeugspitze (10) im zusammengesetzten Zustand koaxial umgeben.

9. Medizinisches Instrument (1) nach einem der Ansprüche 2 bis 8, **dadurch gekennzeichnet, dass** die Führungsnuten (20) in Axialrichtung um den Verschiebeweg der in die jeweiligen Führungsnuten (20) eingreifenden korrespondierenden Führungsstege (21) länger ausgebildet sind, als die jeweiligen Führungsstege (21).

10. Medizinisches Instrument (1) nach einem der Ansprüche 2 bis 8, **dadurch gekennzeichnet, dass** der Verschiebeweg der jeweiligen Führungsstege (21) innerhalb der korrespondierenden Führungsnuten (20) über die axiale Länge der jeweiligen Führungsnuten (20) begrenzbar ist.

## Claims

1. Medical instrument (1) with a hollow shaft (2), at the proximal end (3) of which a handle (4) is arranged, and at the distal end (5) of which a tool (6) is arranged with two jaw parts (7, 8), wherein at least one jaw part (8) is pivotable relative to the other jaw part (7), wherein a distal end region of the shaft (2) that carries the tool (6) is designed as a tool tip (10) that can be deflected with respect to the longitudinal axis (9) of the shaft (2), and wherein the deflection of the tool tip (10) is effected via a first actuation element (11) mounted axially displaceably in the hollow shaft (2) and operatively connected at the proximal end to the handle (4), and wherein the at least one pivotable jaw part (8) of the tool (6) is adjustable between a closed and an opened position via a second actuation element (13) mounted axially displaceably in the hollow shaft (2) and operatively connected at the proximal end to the handle (4), wherein the axially displaceable first actuation element (11) for deflecting the tool tip (10) and the axially displaceable second actuation element (13) for actuating the at least one pivotable jaw part (8) of the tool (6) are arranged parallel to each other in the direction of the longitudinal axis (9) of the shaft (2), **characterized in that** the axially displaceable first actuation element (11) for deflecting the tool tip (10) and the axially displaceable second actuation element (13) for actuating the at least one pivotable jaw part (8) of the tool (6) are mounted to be guided on each other in the axial direction of the longitudinal axis (9) of the shaft (2), and at least one of the two actuation elements (11 or 13) is additionally mounted to be guided on the inner side (18) of the distal end (5) of the shaft (2), wherein the mutual axial mounting of the actuation elements (11 and 13) on each other and also on the inner side (18) of the distal end (5) of the shaft (2) is effected via a pin-and-slot control (19).

2. Medical instrument (1) according to Claim 1, **characterized in that** the pin-and-slot control (19) is designed as a guide groove (20) formed on at least one of the components mounted on each other, the first actuation element (11), the second actuation element (13) or the inner side (18) of the distal end (5) of the shaft (2), and as a guide web (21) formed on the respectively corresponding other component (11, 13 or 18), wherein the guide web (21) is received with guiding in the corresponding guide groove (20).

3. Medical instrument (1) according to Claim 1 or 2, **characterized in that**, in addition to the axially displaceable first actuation element (11) for deflecting the tool tip (10) and the axially displaceable second actuation element (13) for actuating the at least one pivotable jaw part (8) of the tool (6), a rotatably mounted third actuation element (15) for rotating the tool (6) about the longitudinal axis (9) of the shaft (2) is arranged in the hollow shaft (2).

4. Medical instrument (1) according to one of Claims 1 to 3, **characterized in that**, in addition to the axially displaceable first actuation element (11) for deflecting the tool tip (10) and the axially displaceable second actuation element (13) for actuating the at least one pivotable jaw part (8) of the tool (6), and also the third actuation element (15) for rotating the tool (6) about the longitudinal axis (9) of the shaft (2), an axially displaceable fourth actuation element (16) for releasing a jaw part latch (17) is arranged in the shaft (2).

5. Medical instrument (1) according to Claim 4, **characterized in that** the additional axially displaceable fourth actuation element (16) for releasing the jaw part latch (17) is likewise mounted to be guided on the components mounted with guiding on each other, the first actuation element (11), the second actuation element (13) or the inner side (18) of the distal end (5) of the shaft (2).

6. Medical instrument (1) according to Claim 4 or 5, **characterized in that** the first actuation element (11) for deflecting the tool tip (10) is arranged centrally between the second actuation element (13) for actuating the at least one pivotable jaw part (8) and the fourth actuation element (16) for releasing the jaw part latch (17).

7. Medical instrument (1) according to Claim 6, **characterized in that**, in order to form the pin-and-slot control (19), two guide webs (21) are arranged offset by 180° relative to each other on the first actuation element (11) for deflecting the tool tip (10), which guide webs (21) engage in corresponding guide grooves (20) which are formed on the second actuation element (13) for actuating the at least one pivotable jaw part (8) and on the fourth actuation element (16) for releasing the jaw part latch (17), and **in that** a respective guide web (21) is arranged on those sides, facing toward the inner side (18) of the distal end (5) of the shaft (2), of the second actuation element (13) for actuating the pivotable jaw part (8) and of the fourth actuation element (16) for releasing the jaw part latch (16), said respective guide web (21) engaging in a corresponding guide groove (20) which is formed on the inner side (18) of the distal end (5) of the shaft (2).

8. Medical instrument (1) according to Claim 6 or 7, **characterized in that** a shaft (11a) arranged proximally behind the pin-and-slot control (19) and belonging to the first actuation element (11) for deflecting the tool tip (10) is designed with a circular cross section, and the parallel shafts (13a and 16a) of the second actuation element (13) for actuating the at least one pivotable jaw part (8) and of the fourth actuation element (16) for releasing the jaw part latch (17) are each designed with a semi-circular cross section, such that, in the assembled state, the shafts (13a and 16a) with the semi-circular cross section coaxially surround the shaft (11a) of the first actuation element (11) for deflecting the tool tip (10) .

9. Medical instrument (1) according to one of Claims 2 to 8, **characterized in that** the guide grooves (20), in the axial direction, are longer than the respective guide webs (21) by the displacement path of the corresponding guide webs (21) engaging in the respective guide grooves (20).

10. Medical instrument (1) according to one of Claims 2 to 8, **characterized in that** the displacement path of the respective guide webs (21) inside the corresponding guide grooves (20) can be limited via the axial length of the respective guide grooves (20).

## Revendications

1. Instrument médical (1) pourvu d'une tige creuse (2) à l'extrémité proximale (3) de laquelle est disposée une poignée (4) et à l'extrémité distale (5) de laquelle est disposé un outil (6) pourvu de deux parties de mâchoire (7, 8), au moins une partie de mâchoire (8) pouvant pivoter par rapport à l'autre partie de mâchoire (7), une zone d'extrémité distale de la tige (2) qui porte l'outil (6) étant conçue comme une pointe d'outil (10) qui peut être pliée par rapport à l'axe longitudinal (9) de la tige (2) et le pliage de la pointe d'outil (10) étant effectué par le biais d'un premier élément d'actionnement (11) qui est monté dans l'arbre creux (2) de manière à pouvoir coulisser axialement et qui est relié fonctionnellement à la poignée (4) du côté proximal et l'au moins une partie de mâchoire pivotante (8) de l'outil (6) pouvant être déplacée entre une position fermée et une position ouverte par le biais d'un deuxième élément d'actionnement (13) qui est monté dans l'arbre creux (2) de manière à pouvoir coulisser axialement et qui est relié fonctionnellement à la poignée (4) du côté proximal, le premier élément d'actionnement (11) pouvant coulisser axialement et destiné à plier la pointe d'outil (10) et le deuxième élément d'actionnement (13) pouvant coulisser axialement et destiné à actionner l'au moins une partie de mâchoire pivotante (8) de l'outil (6) en direction de l'axe longitudinal (9) de la tige (2) étant disposés parallèlement l'un à l'autre,
**caractérisé en ce que**
le premier élément d'actionnement (11) pouvant coulisser axialement et destiné à plier la pointe d'outil (10) et le deuxième élément d'actionnement (13) pouvant coulisser axialement et destiné à actionner l'au moins une partie de mâchoire pivotante (8) de l'outil (6) dans la direction axiale de l'axe longitudinal (9) de la tige (2) sont montés de manière à être guidés l'un sur l'autre et l'un au moins des deux éléments d'actionnement (11 ou 13) est également monté de manière à être guidés sur le côté intérieur (18) de l'extrémité distale (5) de la tige (2), le montage axial mutuel des éléments d'actionnement (11 et 13) l'un sur l'autre et du côté intérieur (18) de l'extrémité distale (5) de la tige (2) par le biais d'une commande à pivot et rainure (19) étant réalisé.

2. Instrument médical (1) selon la revendication 1, **caractérisé en ce que** la commande à pivot et rainure (19) est conçue comme une gorge de guidage (20) ménagée au niveau d'au moins un des composants montés l'un sur l'autre, du premier élément d'actionnement (11), du deuxième élément d'actionnement (13) ou du côté intérieur (18) de l'extrémité distale (5) de la tige (2) et une nervure de guidage (21) formée au niveau de l'autre composant correspondant (11, 13 ou 18), la nervure de guidage (21) venant se loger dans la gorge de guidage correspondante (20) de manière à être guidée.

3. Instrument médical (1) selon la revendication 1 ou 2, **caractérisé en ce que**, en plus du premier élément d'actionnement (11) pouvant coulisser axialement et destiné à plier la pointe d'outil (10) et du deuxième élément d'actionnement (13) pouvant coulisser axialement et destiné à actionner l'au moins une partie de mâchoire pivotante (8) de l'outil (6), un troisième élément d'actionnement (15) monté à rotation et destiné à faire tourner l'outil (6) sur l'axe longitudinal (9) de la tige (2) est disposé dans la tige creuse (2).

4. Instrument médical (1) selon l'une des revendications 1 à 3, **caractérisé en ce que**, en plus du premier élément d'actionnement (11) pouvant coulisser axialement et destiné à plier la pointe d'outil (10) et du deuxième élément d'actionnement (13) pouvant coulisser axialement et destiné à actionner l'au moins une partie de mâchoire pivotante (8) de l'outil (6) et du troisième élément d'actionnement (15) destiné à faire tourner l'outil (6) sur l'axe longitudinal (9) de la tige (2), un quatrième élément d'actionnement (16) pouvant coulisser axialement et destiné à libérer un doigt d'encliquetage de partie de mâchoire (17) est disposé dans la tige (2).

5. Instrument médical (1) selon la revendication 4, **caractérisé en ce que** le quatrième élément d'actionnement supplémentaire (16) pouvant coulisser axialement destiné à libérer le doigt d'encliquetage de partie de mâchoire (17) est également monté de manière à être guidé sur les composants montés à être guidés, le premier élément d'actionnement (11), le deuxième élément d'actionnement (13) ou le côté intérieur (18) de l'extrémité distale (5) de la tige (2).

6. Instrument médical (1) selon la revendication 4 ou 5, **caractérisé en ce que** le premier élément d'actionnement (11) destiné à plier la pointe d'outil (10) est situé au centre entre le deuxième élément d'actionnement (13) destiné à actionner l'au moins une partie de mâchoire pivotante (8) et le quatrième élément d'actionnement (16) destiné à libérer le doigt d'encliquetage de partie de mâchoire (17).

7. Instrument médical (1) selon la revendication 6, **caractérisé en ce que**, pour former la commande à pivot et rainure (19), deux nervures de guidage (21) décalées de 180° l'une de l'autre sont disposées sur le premier élément d'actionnement (11) destiné à plier la pointe d'outil (10), lesquelles nervures de guidage s'engagent dans des gorges de guidage correspondantes (20) qui sont ménagées au niveau du deuxième élément d'actionnement (13) destiné à actionner l'au moins une partie de mâchoire pivotante (8) et au niveau du quatrième élément d'actionnement (16) destiné à libérer le doigt d'encliquetage de partie de mâchoire (17) et **en ce qu'**une nervure de guidage (21), qui s'engage dans une gorge de guidage correspondante (20) qui est ménagée du côté intérieur (18) de l'extrémité distale (5) de la tige (2), est formée sur les côtés, dirigés vers le côté intérieur (18) de l'extrémité distale (5) de la tige (2), du deuxième élément d'actionnement (13) destiné à actionner la partie de mâchoire pivotante (8) et du quatrième élément d'actionnement (16) destiné à libérer le doigt d'encliquetage de partie de mâchoire (16) .

8. Instrument médical (1) selon la revendication 6 ou 7, **caractérisé en ce qu'**une tige (11a) du premier élément d'actionnement (11) destiné à plier la pointe d'outil (10), laquelle tige est disposée du côté proximal derrière la commande à pivot et rainure (19), a une section transversale circulaire et les tiges (13a et 16a), s'étendant en parallèle, du deuxième élément d'actionnement (13) destiné à actionner l'au moins une partie de mâchoire pivotante (8) et du quatrième élément d'actionnement (16) destiné à libérer le doigt d'encliquetage de partie de mâchoire (17) sont chacune conçues avec une section transversale semi-circulaire de sorte que les tiges (13a et 16a), qui sont de section transversale semi-circulaire, entourent coaxialement la tige (11a) du premier élément d'actionnement (11) destiné à plier la pointe d'outil (10) à l'état assemblé.

9. Instrument médical (1) selon l'une des revendications 2 à 8, **caractérisé en ce que** les gorges de guidage (20) ont par rapport aux nervures de guidage respectives (21) une longueur dans la direction axiale qui est augmentée du chemin de coulissement des nervures de guidage correspondantes (21) s'engageant dans les gorges de guidage respectives (20).

10. Instrument médical (1) selon l'une des revendications 2 à 8, **caractérisé en ce que** le chemin de coulissement des nervures de guidage respectives (21) à l'intérieur des gorges de guidage correspondantes (20) sur la longueur axiale des gorges de guidage respectives (20) peut être limité.
